# EUROPÄISCHE PATENTANMELDUNG

(11) **EP 0 983 766 A1**
(43) Veröffentlichungstag der Anmeldung: **08.03.2000**
(21) Anmeldenummer: 99100923.4
(22) Anmeldetag: 20.01.1999
(51) Int. Cl.: A61K 35/78, A61K 33/34, A61K 33/04

(54) **Pharmazeutische Zusammensetzung zur Behandlung von rheumatischen Syndromen enthaltend Schwefel, Senfsamen und einen Kupfersalz**

(71) Anmelder: Gschwend, Norbert A., 9100 Herisau (CH); Gschwend, Norbert, 9100 Herisau (CH)
(72) Erfinder: Gschwend, Norbert A., 9100 Herisau (CH); Gschwend, Norbert, 9100 Herisau (CH)
(74) Vertreter: Troesch Scheidegger Werner AG

(57) **Zusammenfassung**

Eine pharmazeutische Zusammensetzung zur Behandlung von rheumatischen Syndromen, wie insbesondere Rheuma, Arthritis, Ischias und/oder Gicht ist durch mindestens die Wirkstoffe Schwefel, Senfsamen sowie ein Kupfersalz gekennzeichnet. Weiter kann die pharmazeutische Zusammensetzung optional Kamille, Kampfer und/oder Kaliumiodat enthalten. Als Trägersubstanz wird vorzugsweise Talk verwendet. Die Zusammensetzung liegt vorzugsweise in Form eines Pulvers vor, wie beispielsweise eines Fusspulvers. Aber auch andere Verabreichungsformen, wie beispielsweise Cremen, Salben und dergleichen, sind möglich.

## Beschreibung

Die vorliegende Erfindung betrifft eine pharmazeutische Zusammensetzung mit stark verbesserten Eigenschaften bei der Behandlung von rheumatischen Syndromen, wie insbesondere von Rheuma, Arthritis, Ischias und/oder Gicht, eine kutane Verabreichungsform, enthaltend eine pharmazeutische Zusammensetzung, ein Fusspulver sowie ein Verfahren zur Herstellung einer pharmazeutischen Zusammensetzung bzw. eines Fusspulvers.

Aus der Literatur sind mehrere Wirkstoffe für die Behandlung von rheumatischen Syndromen bekannt. So beschreibt H. Seliger, Sonderdruck "Der praktische Arzt" aus "Zeitschrift für ärztliche Fortbildung", XIII. Jahrgang, 15.11.1959, Nr. 150, Seiten 798 bis 802, dass sich besonders kolloider Schwefel bewährt hat bei der Behandlung von unter anderem Rheuma, Arthritis und Ischias. Als wirkungsvolle Ergänzungen werden Kampfer und Kamillenblüten erwähnt. Insbesondere nimmt H. Seliger Bezug auf ein pharmazeutisches Präparat der Firma N. Gschwend, Herisau, in welchem die drei genannten Wirkstoffe zusammen mit Talk als Trägersubstanz enthalten sind.

Aus dem IKS-Monatsbericht 12/1994 sind in der Monographieliste D, IT07.10.4 Senfsamen und Kamillenblüten als pharmazeutische Wirksubstanzen bei der kutanen Behandlung von Arthritis und rheumatischen Krankheiten bekannt.

Als schwefelhaltige Präparate mit der Indikation "Rheuma" wiederum sind eine Reihe von Badeölen und Badezusätzen bekannt, wie "Soufrol", Schwefel-Öl-Bad und "Leukona" Sulfomoor-Bad.

Die vorliegende Erfindung bezweckt die Schaffung einer weiteren pharmazeutischen Zusammensetzung mit guten bzw. verbesserten Eigenschaften bei der Behandlung von rheumatischen Syndromen.

Erfindungsgemäss wird eine pharmazeutische Zusammensetzung zur Behandlung von rheumatischen Syndromen, wie insbesondere Rheuma, Arthritis, Ischias und/oder Gicht vorgeschlagen, welche mindestens als Wirkstoffe Schwefel, Senfsamen sowie ein Kupfersalz enthält.

Weitere bevorzugte pharmazeutische Zusammensetzungen sind in abhängigen Ansprüchen charakterisiert.

Weiter vorgeschlagen wird eine kutane Verabreichungsform für die Behandlung von rheumatischen Syndromen, enthaltend eine erfindungsgemäss vorgeschlagene pharmazeutische Zusammensetzung. Vorzugsweise handelt es sich bei der vorgeschlagenen kutanen Verabreichungsform um ein feines Fusspulver, speziell ausgerichtet für die Applikation über die Fusssohle.

Das bevorzugt vorgeschlagene feine Fusspulver wird in Schuhe, Socken, Strümpfe oder in Füsslinge eingestreut, und die Absorption der Wirkstoffe erfolgt dann über die Fusssohlen in die Blutbahn. Diese Applikation eines Rheumamittels ist einzigartig und steht in direktem Zusammenhang mit der speziellen erfindungsgemäss vorgeschlagenen Kombination der einzelnen Wirkstoffe. Der Wirkungsmechanismus besteht darin, dass beim Kontakt mit lebendem und verhorntem Gewebe (eben die Fusssohle), auch unter der Einwirkung der natürlichen Aspiration bzw. des natürlichen Schweisses, eine Reihe von chemischen Umsetzungen in Gang gesetzt werden und zu entsprechenden Reaktionen im doppelten Sinne führen - einmal über die Blutbahn sowie auch über das Nervensegment. Voraussetzung hierzu ist selbstverständlich, dass die Wirksubstanzen von der Haut gut resorbiert werden, was durch die erfindungsgemäss vorgeschlagene Kombination von Wirkstoffen sichergestellt ist. Durch die schon vorgängig beschriebene transdermale Absorption nimmt der Organismus nur gerade soviel Wirkstoff auf, wie er wirklich benötigt.

In den erfindungsgemäss vorgeschlagenen Zusammensetzungen ist insbesondere auf die lediglich in Spuren vorhandenen Wirksubstanzen wie das Kupfersalz, vorzugsweise Kupfersulfat, wie auch Kaliumiodat hinzuweisen, welchen offenbar eine gewisse katalytische Wirkungsweise zufällt. So bilden dann diese beiden Substanzen zusammen mit der Trägersubstanz, dem Talk, ein sogenanntes "Katalytpulver", welches den übrigen Wirksubstanzen, wie insbesondere Schwefel und Senfsamen, in kleinsten Mengen hinzugefügt wird.

Bei der Herstellung der pharmazeutischen Zusammensetzung wird zunächst in einem ersten Prozessschritt eine Mischung zwischen Talk und der Wirksubstanz Schwefel sowie optional ggf. Kampfer und Kamillenblüten hergestellt. Dabei erfolgt das Mischen durch bereits vorab hergestellte pulverförmige Wirksubstanzen, wobei selbstverständlich auch der Trägerstoff - das Talk, oder auch genannt Talkum - in Pulverform vorliegt.

In einer zweiten Prozessstufe wird dann dieser Mischung das obengenannte sogenannte "Katalytpulver" beigemengt, wobei lediglich eine Kleinstmenge hinzugefügt wird. Das Katalytpulver besteht wiederum aus Talk sowie Senfsamen, dem Kupfersalz, wie vorzugsweise Kupfersulfat und optional Kaliumiodat.

Der Vorteil des Hinzufügens von Kaliumiodat liegt insbesondere auch darin, die pharmazeutische Zusammensetzung in heissen bzw. tropischen Zonen zu stabilisieren. Bekanntlich ist die Trägersubstanz Talk nicht unbedingt stabil bzw. geeignet für den Gebrauch in tropischen bzw. heissen Gebieten, was das Hinzufügen von Kaliumiodat notwendig bzw. sinnvoll macht.

Die Erfindung wird nun anschliessend anhand der beigefügten Beispiele zur Herstellung und anhand eines Rezepturbeispiels näher erläutert.

Wie bereits oben erwähnt, ist die Rezeptur zweiteilig, d.h. die erfindungsgemäss vorgeschlagene pharmazeutische Zusammensetzung wird in zwei Prozessschritten hergestellt, womit die Rezeptur in zwei sogenannte Tranchen aufgeteilt ist:

| Tranche 1: |
|---|
| Schwefel: ungefähr 30 - 50 Gew.%, vorzugsweise 30 - 40 Gew.%, |
| Kamillen: 0 - 10 %, vorzugsweise 5 - 10 % |
| Kampfer: 0 - 25 %, vorzugsweise 15 - 25 % |
| Talk (Rest) : 20 - 65 % |
| Total Tranche 1: 85 - 95 % |

| Tranche 2: |
|---|
| Senfsamen: 0,5 - 2,5 %, vorzugsweise 1 - 1,5 % |
| Kupfersulfat: 0,05 - 0,3 %, vorzugsweise 0,1 - 0,15 % |
| Kaliumiodat: 0 - 0,15 %, vorzugsweise 0,05 - 0,1 % |
| Talk (Rest): 3 - 13 % |
| Total Tranche 2: 5 - 15 % |

Die Gewichtsprozentangaben beziehen sich auf das Gesamtgewicht der Rezeptur, resultierend aus Tranche 1 und Tranche 2.

Die Herstellung erfolgt, indem zunächst die Mischung der Tranche 1 erstellt wird, wobei zunächst die verschiedenen Komponenten zu Feinstpulver gemahlen und gesiebt werden und dann zusammen mit Talk in einem Mischer gemischt werden, wie beispielsweise in einem sogenannten 4-Weg-Mischer während ca. 15 Min.

Die Mischung der Tranche 2 wird hergestellt, indem zunächst Kupfersulfat und gegebenenfalls Kaliumiodat in einer Reibschale mit Pistill solange verrieben werden, bis ein homogenes Pulver vorliegt. Anschliessend werden diese Komponenten zusammen mit Talk und Senfsamen gesiebt, wie beispielsweise in einem 0,6 mm Sieb und zur Mischung der Tranche 1 gegeben und mit dieser erneut gemischt. Wiederum kann dies in einem 4-Weg-Mischer während ca. 20 min. geschehen.

Selbstverständlich handelt es sich bei den obigen Angaben um Beispielsangaben, welche je nach Applikation bzw. Verabreichungsform, zu behandelndem Leiden, etc. variiert bzw. abgeändert werden können. Auch das Herstellen der Mischungen kann selbstverständlich abweichend von dem oben angeführten Herstellverfahren erfolgen. Hingegen ist es insbesondere bei der Herstellung eines Fusspulvers wichtig, dass die verschiedenen Komponenten bei der Herstellung des Fusspulvers ausgiebig gemischt werden, um ein schlussendlich feines Pulvergemisch zu erhalten.

Als weitere Verabreichungsformen sind auch Cremen, Pasten und dergleichen möglich, in welchen die pharmazeutische Zusammensetzung beispielsweise in Feinstpulverform zusammen mit Trägersubstanzen und weiteren Additiven enthalten ist.

In Ergänzung zu den bereits eingangs erwähnten Indikationen hat es sich gezeigt, dass die erfindungsgemäss vorgeschlagenen pharmazeutischen Zusammensetzungen auch geeignet sind für die Anwendung bei den nachfolgend angeführten Leiden bzw. Krankheiten:

Ischias, Muskelrheumatismus, Arthritis, Phlebitis (Venenentzündung), zu hoher oder zu tiefer Blutdruck, Paralysis deformans, Paralysis post Myelitis, Poliomyelitis, Paralysis cerebralis, Paralysis post Nephritis vel Uraemia, Paralysis postlaesion cause alicuia mechanica, Ekzeme und Röntgenverbrennungen.

## Patentansprüche

1. Pharmazeutische Zusammensetzung zur Behandlung von rheumatischen Syndromen, wie insbesondere Rheuma, Arthritis, Ischias und/oder Gicht, gekennzeichnet durch mindestens die nachfolgenden Wirkstoffe:
Schwefel, Senfsamen sowie ein Kupfersalz.

2. Pharmazeutische Zusammensetzung, insbesondere nach Anspruch 1, dadurch gekennzeichnet, dass als Kupfersalz Kupfersulfat verwendet wird.

3. Pharmazeutische Zusammensetzung, insbesondere nach einem der Ansprüche 1 oder 2, weiter enthaltend Kamille, wie vorzugsweise Kamillenblüten.

4. Pharmazeutische Zusammensetzung, insbesondere nach einem der Ansprüche 1 bis 3, enthaltend Talk als Trägersubstanz.

5. Pharmazeutische Zusammensetzung, insbesondere nach einem der Ansprüche 1 bis 4, weiter enthaltend Kampfer.

6. Pharmazeutische Zusammensetzung, insbesondere nach einem der Ansprüche 1 bis 5, weiter enthaltend Kaliumiodat.

7. Pharmazeutische Zusammensetzung, insbesondere nach einem der Ansprüche 1 bis 5, dadurch gekennzeichnet, dass die Zusammensetzung in Form eines Pulvers vorliegt.

8. Pharmazeutische Zusammensetzung, insbesondere nach einem der Ansprüche 1 bis 7, gekennzeichnet durch folgende mengenmässige Anteile der verschiedenen Bestandteile:
Schwefel: 30 - 50 Gew.%, vorzugsweise 30 - 40 Gew.%
Kamillen: 0 - 10 Gew.%, vorzugsweise 5 - 10 Gew.%
Kampfer: 0 - 25 Gew.%, vorzugsweise 15 - 25 Gew.%
Senfsamen: 0,5 - 2,5 Gew.%, vorzugsweise 1 - 1,5 Gew.%
Kupfersulfat: 0,05 - 0,3 Gew.%, vorzugsweise 0,1 - 0,15 Gew.%
Kaliumiodat: 0 - 015 Gew.%, vorzugsweise 0,05 - 0,1 Gew.%
und der Rest bis 100 Gew.% Talk.

9. Kutane Verabreichungsform, enthaltend eine pharmazeutische Zusammensetzung nach einem der Ansprüche 1 bis 8.

10. Kutane Verabreichungsform, insbesondere nach Anspruch 9, dadurch gekennzeichnet, dass es sich um ein Fusspulver handelt, geeignet für die Applikation über die Fusssohle.

11. Verfahren zur Herstellung einer pharmazeutischen Zusammensetzung nach einem der Ansprüche 1 bis 9, dadurch gekennzeichnet, dass zunächst in einem ersten Schritt Talk und Schwefel in Pulverform gemischt werden und anschliessend in einem zweiten Schritt sogenanntes "Katalytpulver" in kleinen Mengen beigefügt wird, wobei es sich beim Katalytpulver um eine Pulvermischung zwischen Talk, Senfsamen und einem Kupfersalz, wie insbesondere Kupfersulfat, handelt.

12. Verfahren, insbesondere nach Anspruch 11, dadurch gekennzeichnet, dass in der ersten Stufe optional Kampfer und/oder Kamille in Form von Kamillenblütenpulver hinzugefügt werden und dass dem "Katalytpulver" vor Hinzufügen im zweiten Schritt ebenfalls Kaliumiodat beigefügt wird.

13. Verfahren, insbesondere nach einem der Ansprüche 11 oder 12, dadurch gekennzeichnet, dass die Bestandteile in der ersten Stufe zunächst in Pulverform in einem Mischer, wie beispielsweise einem 4-Weg-Mischer, gemischt werden und anschliessend die im zweiten Schritt hinzuzufügenden Bestandteile gesiebt und der Mischung der ersten Stufe in Pulverform hinzugefügt werden und zusammen intensiv gemischt werden.

14. Anwendung des Verfahrens nach einem der Ansprüche 11 bis 13 zur Herstellung eines Fusspulvers für die Behandlung von rheumatischen Syndromen, wie insbesondere Rheuma, Arthritis, Ischias und/oder Gicht.

15. Verwendung der pharmazeutischen Zusammensetzung nach einem der Ansprüche 1 bis 8 zur Behandlung insbesondere einer der nachfolgend angeführten Leiden bzw. Krankheiten:
Ischias, Muskelrheumatismus, Arthritis, Phlebitis (Venenentzündung), zu hoher oder zu niedriger Blutdruck, Paralysis deformans (degenerative, nicht akut entzündliche Erkrankung eines Gelenkes als chronisches Leiden), Paralysis post Myelitis (Entzündung des Rückenmarks), Poliomyelitis (Kinderlähmung), Paralysis cerebralis (Lähmungen das Hirn betreffend), Paralysis post Nephritis vel Uraemia (Lähmung nach Nierenentzündung oder Harnvergiftung), Paralysis postlaesion cause alicuia mechanica (Lähmung nach Verletzungen/Schädigungen nach Operationen, Fall, Schlag, usw.), Ekzeme und/oder Röntgenverbrennungen.
